# EUROPÄISCHE PATENTSCHRIFT

(11) **EP 4 076 604 B1**
(45) Veröffentlichungstag und Bekanntmachung des Hinweises auf die Patenterteilung: **08.05.2024**
(21) Anmeldenummer: 20842174.3
(22) Anmeldetag: 17.12.2020
(51) Int. Cl.: A61M 13/00, A61B 18/00

(54) **GEHÄUSEANORDNUNG EINES RAUCHGASFILTRATIONSSYSTEMS MIT EINER INTEGRIERTEN MÖGLICHKEIT ZUR FLÜSSIGKEITSABSCHEIDUNG**
HOUSING ARRANGEMENT OF A FLUE GAS FILTRATION SYSTEM HAVING AN INTEGRATED OPTION FOR LIQUID SEPARATION
AGENCEMENT DE BOÎTIER D'UN SYSTÈME DE FILTRATION DE GAZ DE FUMÉE AYANT UNE OPTION INTÉGRÉE POUR LA SÉPARATION DE LIQUIDE

(30) Priorität: 18.12.2019 DE 102019008798; 07.05.2020 DE 102020002738
(43) Veröffentlichungstag der Anmeldung: 26.10.2022
(73) Patentinhaber: W.O.M. World of Medicine GmbH, 10587 Berlin (DE)
(72) Erfinder: LANGEN, Fabian, 10407 Berlin (DE); KLUGE, Tobias, 12555 Berlin (DE); BISCHOF, Jan, 10719 Berlin (DE); FELS, Esther, 13357 Berlin (DE); CHAMBERS, Carl, 13505 Berlin (DE)
(74) Vertreter: Gulde & Partner
(86) Internationale Anmeldenummer: PCT/DE2020/000338
(87) Internationale Veröffentlichungsnummer: WO 2021/121462

(56) Entgegenhaltungen:
- US-A1- 2013 174 525
- US-A1- 2015 112 246
- US-A1- 2019 366 251

## Beschreibung

### Erfindungsgegenstand

Gegenstand der Erfindung ist ein für chirurgische Eingriffe bestimmtes Schlauchset mit Geräteankopplung, welches in einem einteiligen Gehäuse eine Anordnung von Wasserfalle und Filter beinhaltet.

### Stand der Technik

Typischerweise erfolgt die Aufdehnung von Körperhöhlen im Rahmen minimalinvasiver chirurgischer Eingriffe mittels Kohlenstoffdioxid-Gas (Insufflation). Diese Erfindung ist auch für jedes andere gasförmige Aufdehnungsmedium nutzbar.

Neben rein mechanischen Instrumenten wie Zangen oder Scheren existieren weitere Gruppen chirurgischer Werkzeuge, die das Gewebe mittels bspw. thermischen Effekten manipulieren können. Zu den gängigen Techniken zählt die Elektrochirurgie (auch Hochfrequenz/ HF-Chirurgie), sowie die Anwendungen von Laserstrahlung oder Ultraschall (US). Die genannten Techniken ermöglichen das Schneiden von Gewebe und eine simultane Blutstillung (Koagulation). Durch die thermische Veränderung des Gewebes entstehen chirurgische Rauchgase, die zum einen die Sicht des Chirurgen beeinträchtigen und zum anderen eine Vielzahl toxischer Bestandteile enthalten. Die Abgasfahne ist ein Aerosol und besteht aus abgelösten Zellfragmenten und Kondensationspartikeln, die inklusive der entstandenen Schadgase aus dem Situs abgeführt werden sollten. Es bedarf demnach einer Vorrichtung, welche den erzeugten Rauch absaugt und zugleich die gesundheitsschädigenden Stoffe herausfiltert, bevor die Abluft in den OP-Saal zurückgeführt wird.

Es hat sich gezeigt, dass eine Vorrichtung basierend auf Faser- oder Membranfiltermedien nicht ausreichend ist, um die während einer OP anfallenden Rauchgase zu filtern. Es hat sich herausgestellt, dass eine zusätzliche Flüssigkeitsabscheidung (im weiteren Verlauf Wasserfalle genannt) insbesondere bei minimal-invasiven chirurgischen Eingriffen erforderlich ist. Nur durch die Abscheidung kondensierter und eingesaugter Flüssigkeiten kann die Standzeit von Faser- oder Membranfiltermedien ausreichend sichergestellt werden. Die Nutzung einer von der Geräteankopplung separierten oder externen Wasserfalle ist zunächst materialaufwändig. Besonders nachteilig ist jedoch die erforderliche Positionierung der Wasserfalle entlang des Schlauches und insbesondere in einer definierten Orientierung relativ zur Schwerkraft.

Bislang wurden separate Gehäuse als externe Wasserfallen im Gasweg des Absaugkanals eingefügt. In der technischen Lösung sind Wasserfallen in vielfachen Ausführungen bekannt und wenden das Prinzip der Trennung durch Schwerkraft an (Wasser sinkt, Gas steigt auf). Zudem sind Lösungsansätze bekannt, bei denen absorbierende Medien genutzt werden sowie Gehäuseanordnungen, die beispielsweise durch eine Trennwand Flüssigkeit von den Filtermedien abgrenzen.

Die freie Lage derartiger Wasserfallen ist besonders negativ aufgefallen, da ein Anheben des Schlauchsets (z.B. durch die OP-Schwester) dazu führen kann, dass die Flüssigkeit zum Partikelfilter gelangt. US2015112246 A1 zeigt eine Filterkartusche mit einer Wasserfalle, zur Verbindung mit einem Insufflator.

### Erfindungsgemäße Lösung

Zur Lösung der oben geschilderten Probleme lehrt die vorliegende Erfindung eine Vorrichtung zur Rauch- und Wasserabscheidung gemäß dem Anspruch 1.

Die erfindungsgemäße Vorrichtung besteht aus einem Gehäuse welches mit der Saugpumpe eines Insufflators mechanisch gekoppelt wird. Die Kopplung erfolgt so, dass die Orientierung des Gehäuses feststeht, so dass eine Drehung um die Verbindungsachse ausgeschlossen ist. Gleichwohl erlaubt die Kopplung eine Fluidverbindung zwischen der Saugpumpe und dem Inneren des Gehäuses. Hierfür sind zahlreiche Lösungsmöglichkeiten bekannt, die an dieser Stelle nicht weiter beschrieben werden müssen.

Das Gehäuse enthält ferner mindestens einen zweiten Anschluss zum Anschluss mindestens eines Desufflationschlauches, welcher Gas aus dem Körperinneren eines Patienten absaugt. Patientenseitig enthält der Desufflationschlauch vorzugsweise einen Trokar.

Das Gehäuse enthält mindestens einen fachüblichen Schwebstoffilter zur Entfernung von Partikeln aus dem Gasstrom. Derartige Filter sind dem Fachmann bekannt und handelsüblich, so dass an dieser Stelle keine weiteren Erläuterungen notwendig sind. Vor dem Hauptschwebstofffilter (flach oder gefaltet, bspw. ULPA-Klasse) kann noch ein Vorfilter (flach oder gefaltet, bspw. EPA-Klasse) angeordnet sein.

Mindestens ein Hohlraum des Gehäuses dient als Wasserfalle zur Abscheidung und Sammlung von Wassertröpfchen aus dem Gassstrom. Die Abscheidung erfolgt dabei rein mechanisch, z.B. dadurch, dass der Gasstrom gegen eine Wand geführt wird, an der die Tropfen dann nach unten ablaufen.

Gegebenenfalls können weitere Wassertröpfchen- oder Wasserdampf-Abscheideelemente angeschlossen sein, die weiter unten näher beschrieben werden. Da das Gehäuse der erfindungsgemäßen Vorrichtung das abgeschiedene Wasser sammeln soll muss das Gehäuse flüssigkeitsdicht ausgestaltet sein.

Die Anordnung der Komponenten im Gehäuse erfolgt so, dass der Gassstrom vom Patienten durch den ersten Schlauch in das Gehäuse führt. Im Gehäuse führt der Gasstrom zunächst durch den Hohlraum zum Zweck der Wasserabscheidung. Anschließend wird das Gas durch das Schwebstofffilter zur Saugpumpe geführt.

Die erfindungsgemäße Gehäuselösung weist mehrere Vorteile auf:
- Durch eine Integration in das Gehäuse des Filtersystems wird das Handling (Positionierung der Gehäuse/Schlauch-Einheit (Schlauchset) und korrekter Anschluss am Gerät) vereinfacht und die Wasserfalle selbst wird in ihrer Lage fixiert. Durch die fixierte Orientierung des Gehäuses ist dieses mit dem Gerät in alle Richtungen nicht drehbar verbunden, ein Auskippen etc. ist nicht möglich.
- Im Gegensatz zu einer Wasserfalle, die beispielsweise mittels einer Trennwand die angesammelte Flüssigkeit in einem ausschließlich dafür vorgesehenen Hohlraum abgrenzt, ermöglicht die erfindungsgemäße Lösung, dass die Flüssigkeit die enthaltene Baugruppe mit den Filtermedien (dreidimensional) umgibt. Das im Inneren freie Volumen des Gehäuses wird demnach bestmöglich zur Flüssigkeitsansammlung genutzt.
- Die Maximierung der Flüssigkeitskapazität erfolgt, indem der Übergang zum Partikelfilter bei fixierter Ausrichtung möglichst weit oben und mit Nutzung aller sich ergebender Hohlräume erfolgt. Die Baugruppe zur Aufnahme der Filtermedien ist derart gestaltet, dass der Fluidfüllstand die Medien nicht erreichen kann, bevor das innere freie Volumen fast vollständig ausgenutzt ist. Die Verwendung des gesamten sich bietenden Hohlraumes erfordert, dass das Gehäuse vollständig flüssigkeitsdicht ist.
- Die Integration der Wasserfalle in das Gehäuse erlaubt eine Positionierung der Schlauchanschlüsse sowohl in horizontaler als auch in vertikaler Lage. Bislang treten die Schläuche senkrecht zur Gerätefront aus, was immer wieder zum Knicken und somit einer zumindest teilweisen Okklusion führt. Daraus resultiert wiederum eine verminderte Absaug- und Insufflationsleistung.
- Die Wasserfalle beinhaltet neben der Ausnutzung der Schwerkraft zusätzliche Wirkprinzipien, die die Flüssigkeitsabscheidung oder Kondensation der im abgesaugten Gas enthaltenen Feuchte begünstigen. Beispiele hierfür sind eine Beschleunigung oder Zirkulation der Strömung durch Axial-/ Tangentialzyklone, Nutzung weiterer Massenträgheitseffekte zum Umlenken oder Erzwingen einer definierten Strömung (z.B. 180° Umlenkung, Prallwand, Düsenströmungen), Einbringung von adsorbierenden oder absorbierenden Medien (z.B. Aktivkohle, superabsorbende Polymere, Öltrennvliese), Nutzung von Wirbelwäscher-Prinzipien sowie die Nutzung von Koaleszenzeffekten durch Drahtgewebe, synthetische Gewebe oder Schäume. Auch die Kombinationen mehrerer genannter Prinzipien ist möglich.
- Mögliche fluidische Schnittstellen/Anschlüsse des Gehäuses können unter Beibehaltung der erfindungsgemäßen Gestaltung sein:
   Gaszufuhr zum Patienten (*inflow*);
   Gasabfuhr vom Patienten (*outflow,* auch zur gezielten Entlüftung (*desufflation*) / Rauchgasabsaugung, wobei dieses Lumen auch zur vermehrten Gaszufuhr zum Patienten (*double insufflation*) genutzt werden kann oder als Unterdruck für einen Absaugbehälter, der Flüssigkeiten und Gewebereste aus der Körperhöhle saugt (chirurgischer Sauger);
Druckmesskanal zur Echtzeitüberwachung des Kavitätsdrucks in der Körperhöhle im Patienten.
- Das Gehäuse ist das patientenferne/proximale Ende des Schlauchsets und dient der Weiterführung aller Geräteverbindungen fluidischer (Inflow, Outflow / *Absaugung,* Druckmesskanäle) und elektrischer Art (Gasheizung, Temperatursensorik, etc.). Die Schlauchlumen können daran fest (beispielsweise durch Verklebung) oder lösbar (beispielsweise durch Steckkupplungen oder ähnliches) gekoppelt werden.
- Das Schlauchset bevorzugt ist als Einwegartikel (Disposable) zum einmaligen Gebrauch vorgesehen. Das bedeutet, es erfolgt normalerweise keine Entleerung, Aufbereitung oder Wiederverwendung.
- Variante 1: Das Gehäuse enthält eine Füllstandsanzeige durch mindestens an einer Stelle transparentes Gehäuse mit oder ohne Skala. Füllstandserfassung durch andere Methoden sind ebenfalls erfindungsgemäß, wie z.B. eine kapazitive Füllstanderfassung oder Füllstandsensor am Gerät der z.B. mit Ultraschall die Füllung erfasst.
- Variante 2: Entleerungsmöglichkeit des *(möglicherweise kontaminierten)* Wasserfalleninhaltes zu Verlängerung der Nutzungsdauer durch beispielsweise ein Septum in der Gehäusewand oder einen Verbinder (z.B. einen Anschluss für eine Spritze mit Luer-Anschluss, oder einen herausgeführten Schlauch mit Schlauchklemme) mit Verschlussmöglichkeit etwa durch ein Rückschlagventil.
- Variante 3: Aufbau eines Gehäuses ohne Insufflationskanal zur Verwendung als mehrfach nutzbares "Tagespatienten"-Schlauchset. Mittels eines zusätzlichen, für jeden Patienten erneuerbaren Schlauchs und einer separaten Insufflationsleitung kann die Kassette zur Rauchgasabsaugung im Gerät verweilen und ohne Aufbereitung für mehrere Patienten verwendet werden.

Weitere optionale Merkmale:
- Umschaltmöglichkeit im Gerät zur Nutzung eines weiteren Lumens, z.B. der Absaugleitung, als zusätzliche Insufflations- / Zufuhrleitung *(double insufflation).* So kann ohne ein zusätzliches Lumen die doppelte Gasmenge eingebracht werden.
- Während der diskontinuierlichen Insufflation sind sogenannte Messpausen vorgesehen, also ein Stopp der aktiven Druckregelung. In diesen Messpausen wird die Gaszufuhr unterbrochen und der sich einstellende Gleichgewichtsdruck in der Körperhöhle durch Sensoren im Gerät ermittelt. In diesen Pausen bricht bei entsprechenden Leckagen der Abdominaldruck etwas ein. Über die Absaugleitung können zum Ausgleich der Leckage kleine Gasvolumina des Aufdehnungsmediums als Stabilisierungspulse abgegeben werden. Zur Vermeidung der Rückführung von Wasserfalleninhalt kann die Wasserfalle in dieser Nutzungsform umgangen werden, z.B. mittels einer Schlauchverbindung im Gehäuse. Auch ein passend positionierter Anschlussort, den der Wasserfalleninhalt nicht erreichen kann, ist erfindungsgemäß.

Alternativ kann ein drittes Lumen benutzt werden, das über einen zusätzlichen Anschluss im Gehäuse die Stabilisierungspulse in die Körperhöhle leitet.
- Ist die Nutzung eines separaten Druckmesskanals (sense line) in Form eines dritten oder vierten Lumens der Schlauchverbindung zur Echtzeit-/ dauerhaften Messung des in der Körperhöhle vorhandenen Druckes mit einem Sensor im Gerät vorgesehen, erfolgt dies ohne Beeinträchtigung der erfindungsgemäßen Funktionen der Gehäuseanordnung. Ähnlich ist eine elektrische Verbindung zu einem im Schlauchset oder am patientennahen Ende positionierten Sensors zu sehen, der durch die Gehäuseanordnung geführt werden kann, ohne die erfindungsgemäße Anordnung verändern zu müssen. Die Positionierung der Schnittstellen erfolgt in Bereichen des Gehäuses, die eine direkte Durchleitung des Messkanals/elektrischen Verbindung vom Gerät zum Schlauchlumen ermöglichen, ohne dabei den Filter durchdringen zu müssen. Der Druckmesskanal kann durch das Volumen der Wasserfalle oder an der Außenseite des Gehäuses entlanggeführt werden, muss jedoch gegenüber der Umgebung abgedichtet sein.
- Die erfindungsgemäße Lösung der Gehäuseanordnung ermöglicht, den Füllstand der Wasserfalle durch eine Sensorik im Gerät zu ermitteln (beispielsweise mittels kapazitiver Sensoren). Diese Erkennung ermöglicht wiederum, dass das Gerät rechtzeitig einen Alarm z.B. durch Warntöne oder optische Signalisierung ausgeben kann oder den aktuellen Füllstand auf dem Gerätedisplay anzeigt.
- Mittels einer zusätzlichen Schnittstelle zum Gerät kann der statische Druck in der Wasserfalle über eine Sensorik ermittelt werden. In einer bevorzugten Lösung ist diese Sensorik über Messkanäle mit Sensoren im Gerät verbunden. Diese Messung erlaubt, den bei der Nutzung zunehmenden Differenzdruck über die Filtermedien zu überwachen und rechtzeitig Warntöne auszugeben, bevor die Saugleistung nachlässt oder die Filtermedien vollständig zugesetzt sind.

In einer optionalen Ausführungsform der Erfindung kann das in der Wasserfalle abgeschiedene Wasser zur Befeuchtung des Gasstroms dienen welcher dem Körper zugeführt wird. Hierzu muss die Wasserfalle in Fluidverbindung mit dem Gaszuführschlauch stehen. Damit kein Druckausgleich zwischen Gaszuführschlauch und dem Schlauch zur Saugpumpe erfolgt, empfiehlt es sich, den in dieser Ausführungsform nötigen Flüssigkeitstransport über einen Docht zu realisieren. Der Docht kann beispielsweise aus Baumwollgewebe gefertigt sein. Durch Kapillarkräfte kann Flüssigkeit über den Docht transportiert werden. In dieser Ausführungsform könnte beispielsweise ein Gaszuführschlauch mit Befeuchtungsvorrichtung (wie beispielsweise in WO2020074027A1, EP2806927A1, EP2804649A1, EP3237046A1 beschrieben) außen an der erfindungsgemäßen Vorrichtung vorbeigeführt werden. Über gasdichte Öffnungen der Wasserfalle einerseits und dem Schlauch andererseits kann ein Docht zwischen Wasserfalle und Befeuchtungsmaterial des Schlauches einen Wassertransport von der Wasserfalle zum Gaszufuhrschlauch erlauben. in einer bevorzugten Ausführungsform werden Gaszuführschlauch mit Befeuchtungsmittel und Wasserfalle in einem einzigen Gehäuse untergebracht. Eine solche Ausführungsform und das dadurch bewirkte Recycling des eingesetzten Wassers hat vor allem den Vorteil, dass bei längeren Behandlungsdauern keine Wassernachfüllung des Befeuchtungsschlauches bzw. der Befeuchtungsvorrichtung und auch kein Entleeren der Wasserfalle nötig ist. Zur Vermeidung von Keimübertragungen kann auch ein Filter eingebaut sein.

### Bezugszeichenliste

(1) Gehäusehälfte mit Geräteschnittstellen
(2) Gehäusehälfte mit Schlauchschnittstellen
(3) Kosmetische Gehäusekappe mit Schlauchdurchführung
(4) Baugruppe mit Filtrationsmedien
(5) Funktionselement zur Unterstützung der Flüssigkeitsabscheidung
(6) Gas- und flüssigkeitsdichte Verbindung
(7) nicht zwingend gas- und flüssigkeitsdichte Verbindung
(8) Geräteschnittstelle für Insufflationskanal
(9) Geräteschnittstelle für Desufflations-/Absaugkanal
(10) Geräteschnittstelle für bspw. Differenzdruckmessung [falls nicht benötigt -> gas- und flüssigkeitsdicht verschlossen]
(11) Geräteschnittstelle für bspw. Echtzeit-Druckmesskanal [falls nicht benötigt -> gas- und flüssigkeitsdicht verschlossen ansonsten -> gas- und flüssigkeitsdicht mit (18) verbunden]
(12) Eingangsschnittstelle von (4) für Insufflation [gas- und flüssigkeitsdicht mit (8) verbunden]
(13) Ausgangsschnittstelle von (4) für Desufflation/Absaugung [gas- und flüssigkeitsdicht mit (9) verbunden]
(14) Ausgangsschnittstelle von (4) für Insufflation
(15) Eingangsschnittstelle von (4) für Desufflation/Absaugung
(16) Eingangsschnittstelle von (2) für Insufflation [gas- und flüssigkeitsdicht mit (14) verbunden]
(17) Ausgangsschnittstelle von (2) für Desufflation/Absaugung
(18) Eingangsschnittstelle von (2) für bspw. Echtzeit-Druckmesskanal
(19) Ausgangsschnittstelle von (2) für Insufflation [Anschluss für (24)]
(20) Eingangsschnittstelle von (2) für Desufflation/Absaugung [Anschluss für (24)]
(21) Ausgangsschnittstelle von (2) für bspw. Echtzeit-Druckmesskanal [Anschluss für (24)]
(22) Öffnung in (3) für ein- oder mehrlumigen Schlauch (24)
(23) Maximal erreichbarer Füllstand in der Wasserfalle
(24) Ein- oder mehrlumiger Schlauch
(25) Patientenschnittstelle (nicht dargestellt), bspw. Luer-Konnektor(en)
(26) Insufflationsfilter (flach oder gefaltet)
(27) Zusätzliches Strömungshindernis am Einlass (15)
(28) Vorfilter (flach oder gefaltet), bspw. EPA-Klasse
(29) Hauptschwebstofffilter (flach oder gefaltet), bspw. ULPA-Klasse
(30) Adsorbierendes Medium, bspw. Aktivkohle
(31) Fluidsperre, bspw. PVDF, PTFE
(32) Hauptkörper von (4) zur Aufnahme der Filtrationsmedien (26) bis (31)
(33) Deckel zum gas- und flüssigkeitsdichten Abschluss von (32)
(34) Filter, bspw. Für Differenzdruckmessung über (4)
(35) Dichtungen für Gerät-Schlauchset-Schnittstellen

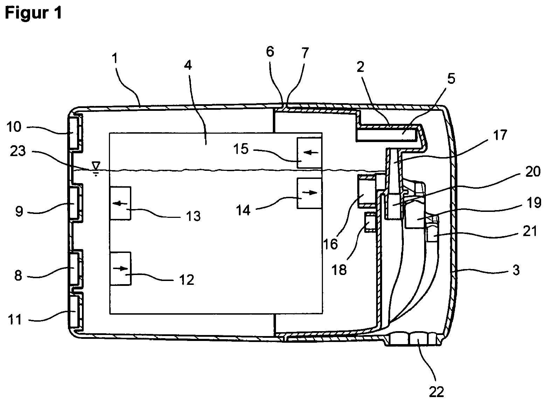

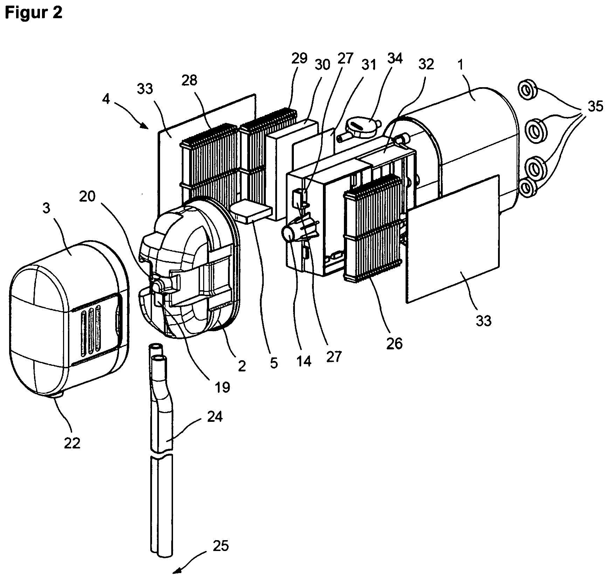

## Patentansprüche

1. Vorrichtung zur Rauch- und Wasserabscheidung eines medizintechnischen Insufflators mit einer Saugpumpe zur Desufflation,
enthaltend
mindestens ein flüssigkeitsdichtes Gehäuse (1,2) mit ersten Anschluss zur mechanischen Kopplung des Gehäuses an das Insufflatorgehäuse, wobei die Kopplung so erfolgt, dass die Orientierung des Gehäuses feststeht und dass eine Drehung um die Verbindungsachse ausgeschlossen ist mindestens ein zweiter Anschluss zum Anschluss mindestens eines Desufflationschlauches,
mindestens ein Schwebstofffilter (29),
mindestens ein Hohlraum,
wobei der Gasstrom vom Patienten durch den ersten Schlauch in das Gehäuse durch den Hohlraum und dann durch das Schwebstofffilter zur Saugpumpe führt,
wobei mindestens ein Hohlraum des Gehäuses als Wasserfalle (4) konfiguriert wird,
wobei der Hohlraum mindestens ein Bauelement zur zusätzlichen Wasserabscheidung enthält, ausgewählt aus:
- Axial- oder Tangentialzyklon,
- Prallwand
- Gasstromumlenkeinrichtung zur Umlenkung des Gasstroms um 90° bis 180°
- Düsen
- adsorbierenden oder absorbierenden Medien
- Wirbelwäscher
- Drahtgewebe-
- synthetische Gewebe oder Schäume.

2. Vorrichtung gemäß Anspruch 1, enthaltend mindestens ein Druckmesskanal (11, 18, 21) zur Echtzeitüberwachung des Kavitätsdrucks in der Körperhöhle des Patienten.

3. Vorrichtung gemäß Anspruch 1 oder 2, enthaltend mindestens eine Füllstandsanzeige.

4. Vorrichtung gemäß einem der Ansprüche 1 bis 3, enthaltend eine verschließbare Öffnung zur Entnahme abgeschiedenen Wassers.

5. Vorrichtung gemäß einem der Ansprüche 1 bis 4, enthaltend mindestens einen Sensor zur Überwachung der verbleibenden Schwebstofffilterkapazität.

## Claims

1. A device for flue gas and water separation of a medical insufflator having a suction pump for desufflation,
including
at least one liquid-tight housing (1, 2) having a first port for mechanically coupling to housing to the insufflator housing, wherein the coupling is carried out such that the orientation of the housing is fixed and that rotation about the connection axis is impossible,
at least one second port for connecting at least one desufflation tube,
at least one particulate filter (29),
at least one cavity,
wherein the gas flow runs from the patient through the first tube into the housing, through the cavity, and then through the particulate filter to the suction pump,
wherein at least one cavity of the housing is configured as a water trap (4),
wherein the cavity includes at least one component for additional water separation, selected from:
- axial or tangential cyclone,
- impact wall
- gas flow deflection system for deflecting the gas flow by 90° to 180°
- nozzles
- adsorbent or absorbent media
- vortex scrubber
- wire mesh
- synthetic mesh or foam.

2. The device according to Claim 1, including at least one pressure-measuring channel (11, 18, 21) for real-time monitoring of the cavity pressure in the body cavity of the patient.

3. The device according to Claim 1 or 2, including at least one fill-level indicator.

4. The device according to any one of Claims 1 to 3, including a sealable opening for removing separated water.

5. The device according to any one of Claims 1 to 4, including at least one sensor for monitoring the remaining capacity of the particulate filter.

## Revendications

1. Dispositif pour la séparation de la fumée et de l'eau d'un insufflateur médical avec une pompe d'aspiration pour la désufflation,
contenant
au moins un boîtier étanche aux liquides (1, 2) avec un premier raccord pour le couplage mécanique du boîtier au boîtier de l'insufflateur, le couplage s'effectuant de telle sorte que l'orientation du boîtier est fixe et qu'une rotation autour de l'axe de liaison est exclue,
au moins un deuxième raccord pour le raccordement d'au moins un tuyau de désufflation,
au moins un filtre pour matières en suspension (29),
au moins une cavité,
le courant de gaz provenant du patient passant par le premier tuyau dans le boîtier, à travers la cavité, puis à travers le filtre pour matières en suspension vers la pompe d'aspiration,
au moins une cavité du boîtier étant configurée sous forme de piège à eau (4),
la cavité contenant au moins un composant pour la séparation d'eau supplémentaire, choisi parmi :
- un cyclone axial ou tangentiel,
- une paroi d'impact,
- un appareil de déviation du courant de gaz pour dévier le courant de gaz de 90° à 180°,
- des buses,
- des milieux adsorbants ou absorbants,
- des laveurs à tourbillon,
- des tissus métalliques,
- des tissus ou mousses synthétiques.

2. Dispositif selon la revendication 1, contenant au moins un canal de mesure de pression (11, 18, 21) pour surveiller en temps réel la pression de la cavité dans la cavité corporelle du patient.

3. Dispositif selon la revendication 1 ou 2, contenant au moins un indicateur de niveau de remplissage.

4. Dispositif selon l'une quelconque des revendications 1 à 3, contenant une ouverture obturable pour prélever l'eau séparée.

5. Dispositif selon l'une quelconque des revendications 1 à 4, contenant au moins un capteur pour surveiller la capacité restante du filtre pour matières en suspension.
